Europäisches Patentamt

(19) European Patent Office (11) Publication number: **0 265 186**

Office européen des brevets **A2**

(12) **EUROPEAN PATENT APPLICATION**

(21) Application number: **87309163.1**

(22) Date of filing: **16.10.87**

(51) Int. Cl.⁴: **A 61 K 7/16**
**A 61 K 7/22**

(30) Priority: **21.10.86 US 921668   05.10.87 US 102721**

(43) Date of publication of application:
**27.04.88   Bulletin   88/17**

(84) Designated Contracting States:
**AT BE CH DE FR GB IT LI LU NL**

(71) Applicant: **THE PROCTER & GAMBLE COMPANY**
**One Procter & Gamble Plaza**
**Cincinnati Ohio 45202  (US)**

(72) Inventor: **White, Donald James, Jr.**
**5690 Bordeaux Way**
**Fairfield Ohio 45014  (US)**

(74) Representative: **Brooks, Maxim Courtney et al**
**Procter & Gamble (NTC) Limited Whitley Road**
**Longbenton**
**Newcastle-upon-Tyne NE12 9TS  (GB)**

(54) **Anticalculus compositions.**

(57) Disclosed are oral compositions containing a mixture of ion-exchanging chelates to provide improved anticalculus activity.

**EP 0 265 186 A2**

Bundesdruckerei Berlin

**Description**

ANTICALCULUS COMPOSITIONS

CROSS REFERENCE TO RELATED APPLICATION

This is a continuation-in-part application of application Serial No. 921,668, filed October 21, 1986.

TECHNICAL FIELD

The present invention relates to oral compositions containing ion-exchanging chelates which provide an improved anticalculus benefit. The preferred chelates include magnesium and strontium EDTA.

BACKGROUND OF THE INVENTION

Dental calculus, or tartar as it is sometimes called, is a deposit which forms on the surfaces of the teeth at the gingival margin. Supragingival calculus appears principally in the areas near the orifices of the salivary ducts; e.g., on the lingual surfaces of the lower anterior teeth and on the buccal surfaces of the upper first and second molars, and on the distal surfaces of the posterior molars.

Mature calculus consists of an inorganic portion which is largely calcium phosphate arranged in a hydroxyapatite crystal lattice structure similar to bone, enamel and dentine. An organic portion is also present and consists of desquamated epithelial cells, leukocytes, salivary sediment, food debris and various types of microorganisms.

As the mature calculus develops, it becomes visibly white or yellowish in color unless stained or discolored by some extraneous agent. This is undesirable from an aesthetic standpoint.

A wide variety of chemical and biological agents have been suggested in the art to retard calculus formation or to remove calculus after it is formed. Mechanical removal of this material pericdically by the dentist is, of course, routine dental office procedure.

The chemical approach to calculus inhibition generally involves chelation of calcium ion and/or crystal growth inhibition which prevents the calculus from forming and/or breaks down mature calculus by removing calcium.

The prior art discloses a number of chelating agents for this purpose. British Patent 490,384, February 15, 1937, discloses oral compositions containing ethylenediaminetetraacetic acid, nitrilotriacetic acid and related compounds as anticalculus agents. U.S. Patent 3,678,154, July 18, 1972 to Widder et al. discloses oral compositions containing certain polyphosphonates and fluoride. U.S. Patent 3,737,533, June 5, 1973 to Francis discloses oral compositions containing certain carbonyl diphosphonates.

In addition to the above references, the prior art discloses dentifrices and mouthwashed containing soluble pyrophosphate salts which have been included for a variety of purposes but not all for anticalculus purposes. Included among such references are U.S. Patent 2,941,926, June 21, 1960 to Salzmann et al. which discloses dental powders containing chlorophyll and pyrophosphate salts. U.S. Patent 3,137,632, June 16, 1964 to Schiraldi discloses toothpastes containing pyrophosphate salts. U.S. Patents 3,927,201 and 202, December 16, 1975 to Baines et al. and Harvey et al., respectively, disclose toothpastes which utilize soluble pyrophosphates as abrasives. U.S. Patents 4,244,931, January 13, 1981 and 4,247,526, January 27, 1981 to Jarvis et al. disclose pyrophosphate salts in dicalcium phosphate systems. Jap. Patent Application Disclosure No 4945-1974 discloses soluble pyrophosphates in a variety of dentifrice systems. U.S. Patent 4,333,551, April 6, 1982 to Parran discloses tetraalkali metal salts in mouthwash compositions. U.S. Patent 4,515,772, May 7, 1985 to Parran, et al., discloses compositions containing soluble pyrophosphate salts as anticalculus agents.

In addition to the use of the above mentioned materials the use of certain acrylic acid polymers and other agents have also been disclosed for use as anticalculus agents. Included among such agents are polyelectrolytes such as copolymers of maleic anhydride and ethylene disclosed in U.S. Patent 3,429,963, February 25, 1969 to Shedlovsky. Shedlovsky also discloses polyacrylic acid having an average molecular weight of 1500 and greater. Another reference disclosing polyacrylic acids in oral compositions is South African Patent 720,898, September 12, 1972 which discloses such acids having a molecular weight in the range of 2,000 to 4,000,000 for use as a membrane to prevent the elution from teeth of previously applied agents. U.S. Patent 3,956,480, May 11, 1976 to Gaffer discloses complexes of anionic polymers (e.g. acrylic acid) and a cationic therapeutic agent (e.g., chlorhexidine) as anticalculus agents. Strontium chelates have also been disclosed for use in oral compositions, particularly in the enhancement of fluoride uptake.

The effect of a strontium-EDTA complex in combination with sodium ricinoleate and a fluoride source is found in the Journal of Dental Research (1982) 61 (3) 451-455. The combined effect of strontium and fluoride in reducing the acid solubility of enamel is also disclosed in the Journal of Dental Research (1983) 62 (10) 1049-1053. A further reference discussing the effect of strontium and fluoride is Featherstone, J.D.B. "Remineralization of Artificial Carious Lesions In-vivo and In-vitro", Proceedings Workshop (1983) IRL Press Ltd.

The use of strontium in combination with fluoride in oral compositions is also disclosed in a number of patent references. Included among these references are U.S. Patent 3,888,976, June 10, 1975 to Mlkvys disclosing an effervescent mouthwash tablet containing strontium ions and possibly a fluoride ion source. U.S. Patent 4,367,219, January 4, 1983 to Schole discloses dentifrices containing a combination of strontium EDTA, a ricinoleate salt and a fluoride ion source. U.S. Patent 4,425,549, November 15, 1983 to Shah et al. discloses toothpastes containing a glycyrrhizinate salt, strontium EDTA and a fluoride ion source. Finally European Patent Application 0,079,611, June 6, 1983, Shah, discloses oral compositions containing a strontium EDTA complex and a fluoride ion source.

Although there have been a number of approaches disclosed for combatting calculus, there is still the desire and need to develop improved products possessing that property. The prior art while disclosing the use of ion exchanging chelates, does not suggest the use of combinations of such agents, let alone combinations of the agents which are claimed herein.

It is an object of the present invention to provide compositions which deliver an anticalculus benefit.

It is a further object of the present invention to produce an effective anticalculus product using a mixture of ion exchanging chelates, which impart increased stability.

It is still a further object of the present invention to provide anticalculus products which are cosmetically acceptable and do not inhibit remineralization of the teeth.

It is still a further object of the present invention to provide effective methods for combating calculus. These and other objects will become more clear from the detailed description which follows.

All percentages and ratios used herein are by weight unless otherwise specified.

## SUMMARY OF THE INVENTION

The present invention embraces an oral composition comprising:
(a) a safe and effective amount of a mixture of two ion exchanging chelates; and
(b) a pharmaceutically acceptable carrier.

The present invention also encompasses a method for retarding development of dental calculus.

## DETAILED DESCRIPTION OF THE INVENTION

The compositions of the present invention comprise a mixture of ion-exchange chelates in a pharmaceutically acceptable carrier.

By "oral compositions" as used herein means a product which in the ordinary course of usage is not intentionally swallowed for purposes of systemic administration of particular therapeutic agents, but is rather retained in the oral cavity for a time sufficient to contact substantially all of the dental surfaces and/or oral tissues for purposes of oral activity.

By "safe and effective amount" as used herein means sufficient amount of material to provide the desired benefit while being safe to the hard and soft tissues of the oral cavity.

By the term "comprising", as used herein, is meant that various additional components can be conjointly employed in the compositions of this invention as long as the listed materials perform their intended functions.

By the term "pharmaceutically acceptable carrier", as used herein, is meant a suitable vehicle which can be used to apply the present anticalculus agents in the oral cavity.

### Ion-Exchanging Chelates

The metal ions found useful in the ion-exchanging chelates include any metal ions that exhibit decreased binding capacity for the chelates relative to calcium. Typical ions include magnesium, strontium and barium. The chelating agents useful are those which effectively bind the metal ions and exhibit increasing binding capacity for calcium relative to the other metal ions. These agents generally have a binding constant, K, the log of which is $\geqq$ 2 (i.e., log K $\geqq$ 2). Suitable chelating agents include: lactic acid, β-hydroxybutyric acid, gluconic acid, malic acid, citric acid, disodium 1, 2-dihydroxybenzene-3, 5-disulfonate, trimetaphosphoric acid, 2-aminobenzoic acid-β, N'-diacetic acid, 3-aminobenzoic acid-N, N-diacetic acid, 4-aminobenzoic acid-N, N-diacetic acid, N-acetamidoiminodiacetic acid, β-(N-trimethylammonium) ethyliminodiacetic acid, N-cyanomethyliminodiacetic acid. N-methoxyethyliminodiacetic acid, aminobarbiteric acid-N, N-diacetic acid, N-hydroxyethyliminodiacetic acid, N-carbethoxy-β-aminoethylimino-diacetic acid, β-aminoethylsulfonic acid-N, N-diacetic acid, β-mercaptoethyliminodiacetic acid, N-methylthioethyliminodiacetic acid, nitrilotriacetic acid, nitrilopropionicdiacetic acid, aminomethylphosphonic acid-N, N diacetic acid, ethylenediamine-N, N-diacetic acid, ethylenediamine-N, N'-diacetic acid, N, N-dihydroxyethylethylenediaminediacetic acid, N-hydroxyethylethylene diaminetriacetic acid, ethylene diaminetetraacetic acid, trimethylene diaminetetracetic acid, tetramethylenediamine tetraacetic acid. pentamethylenediaminetetraacetic acid, 1, 2-diaminocylcohexane-N, N'-tetraacetic acid, 1, 3 diaminocyclohexane-N, N'-tetracetic acid, 1, 2-Bis-[2-dicarboxymethyl-aminoethoxy] ethane, N-hydroxyethyl-N, N', N", N" diethylenetriamine tetraacetic acid, diethylene triaminepentacetic acid... and other, such as those listed in Organic Sequestering Agents, John Wiley & Sons, NY, Stanley Chaberek and Arthur Martell (1957); Organic Ligands, by Douglas D. Perrin, Pergamon Press; and Buffers for pH and Metal Ion Control, D. Perrin and B. Dempsey, Chapman and Hall, London (1974). Preferred agents are EDTA, HEDTA and HIMDA.

The ion exchanging chelates can be put into the compositions as the chelate or formed in-situ when the composition is prepared. In such instances the metal ions can be provided by any of the conventional water soluble salts such as chloride, bromide, nitrate and acetate. The level of the chelated material present in the compositions of the present invention should be sufficient to allow for total metal concentration of from about 0.0243% to about 27.47%, preferably from about 0.0243% to about 6.87% which is sufficient to provide adequate exchangeable metal ion on the tooth and within calculus or plaque.

## Pharmaceutically Acceptable Carrier

The carrier for the active ingredients herein can be any vehicle suitable for use in the oral cavity. Such carriers include the usual components of mouthwashes, dentifrices (e.g., toothpastes, toothpowders, prophylaxis pastes), lozenges, gums and the like and are more fully described hereinafter. Dentifrices and mouthwashes are the preferred systems.

The abrasive polishing material contemplated for use in the dentifrice compositions of the present invention can be any material which does not excessively abrade dentin. These include, for example, silicas including gels and precipitates, calcium carbonate, dicalcium orthophosphate dihydrate, calcium pyrophosphate, tricalcium phosphate, calcium polymetaphosphate, insoluble sodium polymetaphosphate, hydrated alumina, and resinous abrasive materials such as particulate condensation products of urea and formaldehyde, and others such as disclosed by Cooley et al. in U.S. Patent 3,070,510, December 25, 1962, incorporated herein by reference. Mixtures of abrasives may also be used.

Silica dental abrasives, of various types, can provide the unique benefits of exceptional dental cleaning and polishing performance without unduly abrading tooth enamel or dentin. Silica abrasive materials are also exceptionally compatible with sources of soluble fluoride. For these reasons they are preferred for use herein.

The silica abrasive polishing materials useful herein, as well as the other abrasives, generally have an average particle size ranging between about 0.1 and 30 microns, preferably 5 and 15 microns. The silica abrasive can be precipitated silica or silica gels such as the silica xerogels described in Pader et al., U.S. Patent 3,538,230, issued March 2, 1970 and DiGiulio, U.S. Patent 3,862,307, June 21, 1975, both incorporated herein by reference. Preferred are the silica xerogels marketed under the tradename "Syloid" by the W. R. Grace & Company, Davison Chemical Division. Preferred precipitated silica materials include those marketed by the J. M. Huber Corporation under the tradename, "Zeodent", particularly the silica carrying the designation "Zeodent 119". These silica abrasives are described in U.S. Patent 4,340,583, July 29, 1982, incorporated herein by reference.

The abrasive in the compositions described herein is present at a level of from about 6% to 70%, preferably from about 15% to about 25% when the dentifrice is a toothpaste. Higher levels, as high as 90%, may be used if the composition is a toothpowder.

Flavoring agents can also be added to dentifrice compositions. Suitable flavoring agents include oil of wintergreen, oil of peppermint, oil of spearmint, oil of sassafras, and oil of clove. Sweetening agents which can be used include aspartame, acesulfame, saccharin, dextrose, levulose and sodium cyclamate. Flavoring and sweetening agents are generally used in dentifrices at levels of from about 0.005% to about 2% by weight.

Dentifrice compositions can also contain emulsifying agents. Suitable emulsifying agents are those which are reasonably stable and foam throughout a wide pH range, including non-soap anionic, nonionic, cationic, zwitterionic and amphoteric organic synthetic detergents. Many of these suitable surfactants are disclosed by Gieske et al. in U.S. Patent 4,051,234, September 27, 1977, incorporated herein by reference.

It is common to have a water-soluble fluoride compound present in dentifrices in an amount sufficient to give a fluoride concentration of from about 0.0025% to about 5.0% by weight, preferably from about 0.005% to about 2.0% by weight, to provide additional anticaries effectiveness. Preferred fluorides are sodium fluoride, stannous fluoride, indium fluoride, and sodium monofluorophosphate. Norris et al., U.S. Patent 2,946,735, issued July 26, 1960 and Widder et al., U.S. Patent 3,678,154, issued July 18, 1972 disclose such salts as well as others. Both patents are incorporated herein by reference.

Another material useful in the present toothpastes is an anionic anticalculus agent. The anionic calculus inhibitors useful in the compositions of the present invention can be any of the agents which are known to provide calculus reduction. Many of such agents are disclosed in the references listed in the background section herein all of which are incorporated herein by reference. Preferred agents include soluble pyrophosphate salts, soluble polycarboxylic salts (e.g., polyacrylic acid), polyphosphates among many others. The anionic agent is present at a level of from about 0.01% to about 10%, preferably from about 1% to about 5%.

Water is also present in the toothpastes of this invention. Water employed in the preparation of commercially suitable toothpastes should preferably be deionized and free of organic impurities. Water generally comprises from about 10% to 50%, preferably from about 20% to 40%, by weight of the toothpaste compositions herein. These amounts of water include the free water which is added plus that which is introduced with other materials such as with sorbitol.

In preparing toothpastes, it is necessary to add some thickening material to provide a desirable consistency. Preferred thickening agents are carboxyvinyl polymers, carrageenan, hydroxyethyl cellulose and water soluble salts of cellulose ethers such as sodium carboxymethyl cellulose and sodium carboxymethyl hydroxyethyl cellulose. Natural gums such as gum karaya, xanthan gum, gum arabic, and gum tragacanth can also be used. Colloidal magnesium aluminum silicate or finely divided silica can be used as part of the thickening agent to further improve texture. Thickening agents in an amount from 0.2% to 5.0% by weight of the total composition can be used.

It is also desirable to include some humectant material in a toothpaste to keep it from hardening. Suitable humectants include glycerin, sorbitol, and other edible polyhydric alcohols at a level of from about 15% to about 70%.

Another preferred embodiment of the present invention is a mouthwash composition. Conventional mouthwash composition components can comprise the carrier for the agents of the present invention. Mouthwashes generally comprise from about 20:1 to about 2:1 of a water/ethyl alcohol solution and preferably other ingredients such as flavor, sweeteners, humectants and sudsing agents such as those mentioned above for dentifrices. The humectants, such as glycerin and sorbitol give a moist feel to the mouth. Generally, on a weight basis the mouthwashes of the invention comprise 5% to 60% (preferably 5% to 20%) ethyl alcohol, 0% to 20% (preferably 5% to 20%) of a humectant, 0% to 2% (preferably 0.01% to 1.0%) emulsifying agents, 0% to 0.5% (preferably 0.005% to 0.06%) sweetening agent such as saccharin, 0% to 0.3% (preferably 0.03% to 0.3%) flavoring agent, and the balance water. Other optional components described herein earlier for use in dentifrice products are also useful in the mouthwash compositions.

Suitable lozenge and chewing gum components are disclosed in U.S. Patent 4,083,955, April 11, 1978 to Grabenstetter et al., incorporated herein by reference.

The pH of the present compositions and/or its pH in the mouth can be any pH which is safe for the mouth's hard and soft tissues. Such pH's are generally from about 3 to about 10, preferably from about 4 to about 8.

## METHOD OF MANUFACTURE

The compositions of the present invention can be made in a three step process. If magnesium and strontium EDTA are the ion-exchanging chelates in a mouthwash composition, a suitable process is as follows.

### Forming MgEDTA Complex

To part of the water in a composition is added the appropriate amount of $Na_2EDTA$ with mixing until in solution, and then adding the appropriate amount of $MgCl_2$. Mixing is continued until a milky solution occurs which is made clear by addition of caustic to a pH of from 7.1 to 7.3. An acid such as benzoic acid may be used to adjust the pH. This complex solution is then added to the main mix tank.

### Forming SrEDTA Complex

In a separate container an appropriate amount of $Na_2EDTA$ is added to water with mixing until a clear solution is obtained. To this solution is added an appropriate amount of $Sr(OH)_2$. This mixture is then added to the main mix tank.

### Main Mix Tank

The water not used with the complexes is added to a main mix tank. Humectant is then added, premixed with the anionic anticalculus agent, if present, to the tank. A fluoride agent, if present, is then added followed by sweetener, dye and a separate mixture of flavors, alcohol and surfactants.

The present compositions are used in a conventional manner wherein the amounts of product are what users generally use.

The following examples further describe and demonstrate preferred embodiments within the scope of the present invention. The examples are given solely for illustration and are not to be construed as limitations of this invention as many variations thereof are possible without departing from the spirit and scope thereof.

## EXAMPLE 1

Given below is mouthwash representative of the present invention.

| Component | Wt. % |
|---|---|
| Double reverse osmosis water | 68.321 |
| ETOH 190 by weight | 8.500 |
| Sorbitol (70% Aqueous Solution) | 18.00 |
| Tetrasodium pyrophosphate | 1.313 |
| $Na_2EDTA$ | 1.359 |
| $MgCl_2$ 6 $H_2O$ | 0.381 |
| Polysorbate 80 | 0.600 |
| $Sr(OH)_2$ $8H_2O$ | 0.471 |
| Sodium acid pyrophosphate | 0.329 |
| Dye (1% Aqueous Solution) | 0.070 |
| Pluronic F 127[1] | 0.200 |
| Flavor | 0.075 |
| NaSaccharin | 0.040 |
| 50% NaOH | 0.291 |
| Sodium Fluoride | 0.050 |
| | 100.000 |

[1]: A nonionic having ethylene oxide/propylene oxide structure offered by BASF.

EXAMPLE II
Given below is another mouthwash representative of the present invention.

| Component | Wt. % |
|---|---|
| ETOH 190 by weight | 8.500 |
| Sorbitol (70% Solution) | 18.00 |
| Tetrasodium pyrophosphate | 1.671 |
| $Na_2EDTA$ | 0.686 |
| $MgCl_2$  6 $H_2O$ | 0.190 |
| Polysorbate 80 | 0.600 |
| $Sr(OH)_2$ $8H_2O$ | 0.234 |
| Sodium acid pyrophosphate | 0.419 |
| Dye (1% Aqueous Solution) | 0.070 |
| Pluronic F 127[1] | 0.200 |
| Flavor | 0.075 |
| NaSaccharin | 0.040 |
| 50% NaOH | adjust to proper pH |
| Sodium Fluoride | 0.050 |
| Double reverse osmosis water | qs 100% |
| | 100.000 |

[1]: A nonionic having ethylene oxide/propylene oxide structure offered by BASF.

## EXAMPLE III

Given below is another mouthwash representative of the present invention.

| Component | Wt. % |
|---|---|
| ETOH 190 by weight | 8.500 |
| Sorbitol (70% Solution) | 18.00 |
| Tetrasodium pyrophosphate | 1.313 |
| $Na_2EDTA$ | 1.558 |
| $MgCl_2$  6 $H_2O$ | 0.576 |
| Polysorbate 80 | 0.600 |
| $Sr(OH)_2$ $8H_2O$ | 0.359 |
| Sodium acid pyrophosphate | 0.419 |

| | |
|---|---|
| Dye (1% Aqueous Solution) | 0.070 |
| Pluronic F 127[1] | 0.200 |
| Flavor | 0.075 |
| NaSaccharin | 0.040 |
| 50% NaOH | adjust to proper pH |
| Sodium Fluoride | 0.050 |
| Double reverse osmosis water | qs 100% |
| | . 100.000 |

[1]: A nonionic having ethylene oxide/propylene oxide structure offered by BASF.

EXAMPLE IV
Given below is another mouthwash representative of the present invention.

| Component | Wt. % |
|---|---|
| ETOH 190 by weight | 8.500 |
| Sorbitol (70% Aqueous Solution) | 18.00 |
| Tetrasodium pyrophosphate | 1.313 |
| $Na_2EDTA$ | 1.370 |
| $MgCl_2$ 6 $H_2O$ | 0.259 |
| Polysorbate 80 | 0.600 |
| $Sr(OH)_2$ $8H_2O$ | 0.640 |
| Sodium acid pyrophosphate | 0.329 |
| Dye (1% Aqueous Solution) | 0.070 |
| Pluronic F 127[1] | 0.200 |
| Flavor | 0.075 |
| NaSaccharin | 0.040 |
| 50% NaOH | adjust to proper pH |
| Sodium Fluoride | 0.050 |
| Double reverse osmosis water | qs 100% |
| | 100.000 |

[1]: A nonionic having ethylene oxide/propylene oxide structure offered by BASF.

Toothpastes using the components described on pages 6,7 and 8 may also be prepared using the chelates in the above examples.

**Claims**

1. An anticalculus composition comprising a pharmaceutically acceptable carrier characterized in that it further contains a safe and effective amount of a mixture of two ion exchanging chelates wherein the metal ions associated with said chelates are different but the chelating agents may be the same or different and the binding constant of the chelating agent(s) for calcium is greater than for the other metals.

2. An anticalculus composition according to Claim 1 wherein the log of the binding constant of the chelating agents $\geqq 2$.

3. An anticalculus composition according to any one of the preceding claims wherein the metal ion associated with the chelates is selected from the group consisting of strontium, barium and magnesium.

4. An anticalculus composition according to any one of the preceding claims wherein the chelating agents are selected from the group consisting of EDTA, HEDTA, HIMDA and mixtures thereof.

5. An anticalculus composition according to any one of the preceding claims wherein an anionic calculus inhibitor is also present.

6. An anticalculus composition according to Claim 5 wherein the calculus inhibitor is selected from the group consisting of pyrophosphate salts, polyacrylic acid salts and mixtures thereof.

7. An anticalculus composition according to any one of the preceding claims which additionally contains a soluble fluoride source.

8. An anticalculus composition according to any one of the preceding claims which is in the form of a mouthrinse.

9. An anticalculus composition according to any one of the preceding claims which is in the form of a toothpaste.

10. An anticalculus composition according to any one of the preceding claims wherein the chelates are SrEDTA and MgEDTA.

11. An anticalculus composition according to any one of the preceding claims which is in the form of a lozenge.

12. An anticalculus composition according to any one of the preceding claims which is in the form of chewing gum.